Europäisches Patentamt

European Patent Office

Office européen des brevets

(19)

(11) EP 0 848 934 A1

(12) EUROPÄISCHE PATENTANMELDUNG

(43) Veröffentlichungstag:
24.06.1998 Patentblatt 1998/26

(51) Int. Cl.6: **A61B 5/22**, A63B 23/04,
A63B 21/00

(21) Anmeldenummer: 97121871.4

(22) Anmeldetag: 11.12.1997

(84) Benannte Vertragsstaaten:
AT BE CH DE DK ES FI FR GB GR IE IT LI LU MC
NL PT SE
Benannte Erstreckungsstaaten:
AL LT LV MK RO SI

(30) Priorität: 18.12.1996 DE 19652869

(71) Anmelder:
Bavaria Patente und Lizenzen
Verwertungsgesellschaft mbH
01731 Kreischa (DE)

(72) Erfinder:
Blümel, Georg, Doz.Dr.sc.nat.
04209 Leipzig (DE)

(74) Vertreter:
von Samson-Himmelstjerna, Friedrich R., Dipl.-
Phys. et al
SAMSON & PARTNER
Widenmayerstrasse 5
80538 München (DE)

(54) **Kniebeugen-Ergometer**

(57) Die Erfindung schafft ein transportables Knie-
beugen-Ergometer zum Bestimmen der Arbeit und
/oder Leistung einer Testperson. Das erfindungsgemässe Kniebeugen-Ergometer weist Befestigungsmittel
2, 4 zur Befestigung zweier vertikal beabstandeter Messpunkte an oder in Bezug auf eine(r) Testperson derart,
daß der Meßpunktabstand sich durch Beugen / Strekken der Knie ändert; eine Messeinrichtung 3, 6, 8, die
ausgelegt ist: zur Messung der mit dem Beugen und
Strecken der Knie einhergehenden Änderung des
Abstandes zwischen den beiden Messpunkten und /
oder zur Messung der Geschwindigkeit der vorgenannten Abstandsänderung; und eine mit der Messeinrichtung 3, 6, 8 verbundene Auswerteeinrichtung, die
ausgelegt ist: zur Speicherung eines ihr eingegebenen,
der Testperson zugeordneten Gewichtswertes und zur
Ermittlung der von der Testperson erbrachten Arbeit
und / oder Leistung oder einer hierzu korrelierten
Grösse, und zwar durch Verknüpfung des Gewichtswertes mit dem oder den von der Messeinrichtung 3, 6, 8
gelieferten Messwert(en) auf.

Fig. 1

## Beschreibung

Die Erfindung betrifft ein Kniebeugen-Ergometer zur Ermittlung der von einer Testperson beim Beugen und Strecken der Knie erbrachten Arbeit und / oder Leistung oder einer hierzu korrelierten Grösse.

Aus der US 3,861,215 (BRADLEY) ist ein Übungsgerät für Gewichtheber bekannt, bei dem der Übende eine Stange, an der Seile befestigt sind, nach oben stemmt. Die Seile werden durch das Gerät mit einstellbarer Kraft gebremst und auch die Geschwindigkeit, mit der sie frei gegeben werden, kann eingestellt werden, so daß für den Übenden die Wirkung von Gewichten simuliert wird. Das bekannte Übungsgerät ist vergleichsweise groß und sperrig.

Aus der DE 25 40 493 A1 (PUTSCH) ist ein Fahrradergometer mit einem Rechner bekannt, welcher eingegebene Gewichtswerte einer Testperson mit gemessenen Werten (physische Leistung) verknüpft. Auch dieses Gerät ist relativ sperrig und- wie auch das erstgenannte Übungsgerät - nur schwer oder überhaupt nicht von einer Testperson transportierbar.

Aus der US-A-1,308,675 (KELLEY) ist ein Pedomotor zum Unterstützen der Laufbewegung einer Testperson bekannt. Dieser bekannte Pedomotor weist zwei Sätze künstlicher Bänder auf. Diese Bänder sind entsprechend der Anatomie der Beinmuskulatur ausgehend von entsprechenden Festpunkten an den Füssen der Testperson über Gleitführungen mit einer am Oberkörper der Testperson vorgesehenen Zugeinrichtung verbunden. Die Zugeinrichtung wird beim Laufen derart gesteuert, dass sie die jeweiligen Bänder zur Unterstützung der Laufbewegung entsprechend entlastet bzw. belastet.

Die DE-PS-247 482 (FABIANI) offenbart eine Vorrichtung zur Erleichterung des Bergsteigens, welche die von den Streckmuskeln zu leistende Arbeit beim Strecken des im Kniegelenk gebeugten und mit dem Körpergewicht belasteten Beines teilweise übernimmt. Bei dieser bekannten Vorrichtung ist an jedes Bein ein zweiteiliger Lenker angelenkt. Der Lenker ist aus zwei übergreifenden und längs zueinander verschiebbaren Längenabschnitten zusammengesetzt. Beim Biegen des Kniegelenks schieben sich die beiden Lenkerabschnitte zusammen, und beim Strecken wird ein auf dem Rücken der Testperson vorgesehener Arbeitsmechanismus aktiviert, der die beiden Lenkerabschnitte auseinanderschiebt.

In der DE-GM-82 00 338.6 (OMNIPLAST) ist eine Vorrichtung zum Messen der Sprungkraft von Personen offenbart. Diese bekannte Vorrichtung weist eine ebene Sprungplatte mit einem durch eine zentrale Öffnung herausziehbaren, am Körper der springenden Person mittels einer Schnur am vorderen und hinteren Bereich eines Gürtels zu befestigenden Messband auf. Auf der Sprungplatte vorgesehene konzentrische Kreise in Verbindung mit einer Längenskala auf dem Messband werden dabei zur Sprunghöhenermittlung benutzt.

Die US-A-4,848,152 (PRATT) offenbart eine Biofeedback-Hebevorrichtung zum Ermitteln der Hebefähigkeit einer Testperson und Anzeigen der Resultate auf einem Monitor. Bei dieser bekannten Vorrichtung zieht der Testperson an einem Handgriff eines aus einer Bodenplatte ragenden Zugbands. Das Zugband ist mit einem unter der Bodenplatte befindlichen Zugkraft-Erzeugungsmechnismus verbunden, der eine Spule zum Auf- und Abwickeln des Zugbands aufweist, an welche über einen steuerbaren Motor ein vorbestimmtes Drehmoment anlegbar ist. Weiterhin ist eine Umlenkrolle zum Umlenken des Zugbands vorgesehen, die mit Kraftmesseinrichtungen zum Bestimmen der horizontalen und der vertikalen Kraftkomponenten verbunden ist.

Aus der DE 37 44 954 C2 (STRAATEN) ist ein Übungsgerät bekannt, welches eine gegenüber der Horizontalen geneigte Schiene aufweist, längs der ein Gleitbrett zwischen einer oberen, vorderen Lage und einer unteren, hinteren Lage bewegt werden kann. Die Bewegung wird vom Testperson mit einem Seil gesteuert, das mit einem an dem Gleitbrett und an einem Rahmen vorgesehenen Rollensystem verbunden ist.

Die US-A-5,263,913 (BOREN) offenbart ein Übungsgerät, das eine schräg gelagerte Plattform aufweist, die entlang einer Schiene verschiebbar gelagert ist. Die Plattform wird vom Testperson mittels seiner Schultern nach oben und unten bewegt, und die dazu erforderliche Kraft wird aus der Schräglage und dem Gewicht der Plattform bestimmt.

Diesen bekannten Vorrichtungen zur körperlichen Ertüchtigung bzw. zur Unterstützung der Körperkraft ist gemeinsam, dass sie einen aufwendigen, komplizierten Aufbau besitzen, grossteils nur schwer oder überhaupt nicht von ihrem Testperson transportiert werden können und nur schwer installierbar sind.

Die Erfindung zielt darauf ab, ein Ergometer zu schaffen, dessen Konstruktionsprinzip einen einfachen Aufbau und kleine Abmessungen ermöglicht.

Erfindungsgemäss wird obige Aufgabe durch den Gegenstand des Anspruchs 1 gelöst, also durch ein Kniebeugen-Ergometer mit Befestigungsmitteln zur Befestigung zweier vertikal beabstandeter Messpunkte an oder in Bezug auf eine(r) Testperson derart, daß der Meßpunktabstand sich durch Beugen / Strecken der Knie ändert; einer Messeinrichtung, die ausgelegt ist: zur Messung der mit dem Beugen und Strecken der Knie einhergehenden Änderung des Abstandes zwischen den beiden Messpunkten und / oder zur Messung der Geschwindigkeit der vorgenannten Abstandsänderung; und einer mit der Messeinrichtung verbundenen Auswerteeinrichtung, die ausgelegt ist: zur Speicherung eines ihr eingegebenen, der Testperson zugeordneten Gewichtswertes und zur Ermittlung der von der Testperson erbrachten Arbeit und / oder Leistung oder einer hierzu korrelierten Grösse, und zwar durch Verknüpfung des Gewichtswertes mit dem oder den von der Messeinrichtung gelieferten Messwert(en).

Vorteilhafterweise kann bereits das Körpergewicht das Trainingsgewicht bilden. Es können aber auch weiteren wirkenden Kräften, z.B. systemimmanenten oder extern gesteuert erzeugten Gegenkräften, zugeordnete Kraftwerte bei der Ermittlung der erbrachten Arbeit und / oder Leistung berücksichtigt werden.

Bevorzugte Weiterbildungen sind Gegenstand der Unteransprüche.

Gemäss einer bevorzugten Ausführungsform sind die Befestigungsmittel (12) derart angeordnet, dass der erste Messpunkt unterhalb der Knie der Testperson liegt und der zweite Messpunkt oberhalb der Knie der Testperson liegt (Anspruch 2).

Gemäss einer weiteren bevorzugten Ausführungsform sind die Befestigungsmittel an einem ersten Messpunkt, der getrennt vom Körper der Testperson, insbesondere am Fussboden unter der Testperson, vorgesehen ist, befestigbar (Anspruch 3). Mit solch einem Aufbau lassen sich vorteilhafterweise auch Sprungübungen durchführen.

Gemäss einer weiteren bevorzugten Ausführungsform sind die Befestigungsmittel an einem ersten Messpunkt, der am Körper der Testperson, insbesondere am Fuss oder Sprunggelenk der Testperson, vorgesehen ist, befestigbar (Anspruch 4). Dieser Aufbau ist von Vorteil, wenn die Übung durchgeführt werden soll, ohne dass es einer Fixierung der Testperson an einem bestimmten Ort bedarf.

Gemäss einer weiteren bevorzugten Ausführungsform weist die Messeinrichtung eine nach Art eines Seiles, eines Bandes, einer Kette oder dergleichen ausgebildete Zugeinrichtung auf, die den ersten und den zweiten Messpunkt verbindet (Anspruch 5).

Gemäss einer weiteren bevorzugten Ausführungsform weist die Messeinrichtung eine Spuleneinrichtung zum Auf- und Abspulen von zumindest einem Teil der Zugeinrichtung auf (Anspruch 6). Dieser Aufbau ermöglicht eine schnelle und zuverlässige Erfassung der Abstandsänderung und deren Geschwindigkeit.

Gemäss einer weiteren bevorzugten Ausführungsform weist die Messeinrichtung eine Drehwinkel-Erfassungseinrichtung zum Erfassen des Drehwinkels und eine Drehwinkelgeschwindigkeits-Erfassungseinrichtung zum Erfassen der Drehwinkelgeschwindigkeit einer Umlenkeinrichtung für die Zugeinrichtung auf (Anspruch 7). Dieser Aufbau bringt den Vorteil, dass Drehwinkel leicht und genau zu messen sind.

Gemäss einer weiteren bevorzugten Ausführungsform ist die Umlenkeinrichtung die Spuleneinrichtung (Anspruch 8).

Gemäss einer weiteren bevorzugten Ausführungsform ist die Spuleneinrichtung in einem vergleichsweise kleinen Gehäuse untergebracht, welches mittels der Befestigungsmittel am Körper der Testperson anbringbar ist (Anspruch 9). Ein solcher Aufbau schafft ein kompaktes, von der Testperson bequem transportierbares Übungsgerät.

Gemäss einer weiteren bevorzugten Ausführungsform weist die Messeinrichtung eine optische oder eine elektronische Sensoreinrichtung zum Erfassen der Änderung des gegenseitigen Abstands der beiden Messpunkte und der Geschwindigkeit der Abstandsänderung auf (Anspruch 10). Besonders bevorzugt ist eine kontaktlose Sensoreinrichtung wegen ihrer nahezu verschleissfreien Arbeitsweise.

Gemäss einer weiteren bevorzugten Ausführungsform ist die Messeinrichtung mit einem Computer verbunden (Anspruch 11). Der Computer kann insbesondere eine Eingabeeinrichtung sowie eine Speichereinrichtung aufweisen. Dies ermöglicht vorteilhafterweise die Durchführung des sogenannten Biofeedback-Verfahrens, bei dem die vom Testsubjekt unter bestimmten Bedingungen erbrachte Arbeit und / oder Leistung angezeigt und gespeichert und insbesondere auch Vorgaben gemacht werden können. Der Computer kann bei der Auswertung der Messresultate auch zusätzliche physische Parameter der Testperson, wie z.B. Alter, Geschlecht, Grösse usw., berücksichtigen.

Gemäss einer weiteren bevorzugten Ausführungsform ist der Computer ein in dem am Körper der Testperson anbringbaren Gehäuse untergebrachter Mikrocomputer (Anspruch 12). Hierdurch lässt sich ein Stand-Alone-System ohne weitere Komponenten realisieren.

Im folgenden wird die vorliegende Erfindung anhand bevorzugter Ausführungsformen unter Bezugnahme auf die begleitenden Zeichnungen näher erläutert werden.

In den Figuren zeigen:

Fig. 1 eine schematische Darstellung einer ersten bevorzugten Ausführungsform des erfindungsgemässen Kniebeugen-Ergometers;

Fig. 2 eine schematische Darstellung einer zweiten bevorzugten Ausführungsform des erfindungsgemässen Kniebeugen-Ergometers; und

Fig. 3 eine detailliertere schematische Darstellung einer Ausführungsform der Messeinrichtung des erfindungsgemässen Kniebeugen-Ergometers.

In den Figuren bezeichnen gleiche Bezugszeichen gleiche oder funktionsgleiche Bestandteile.

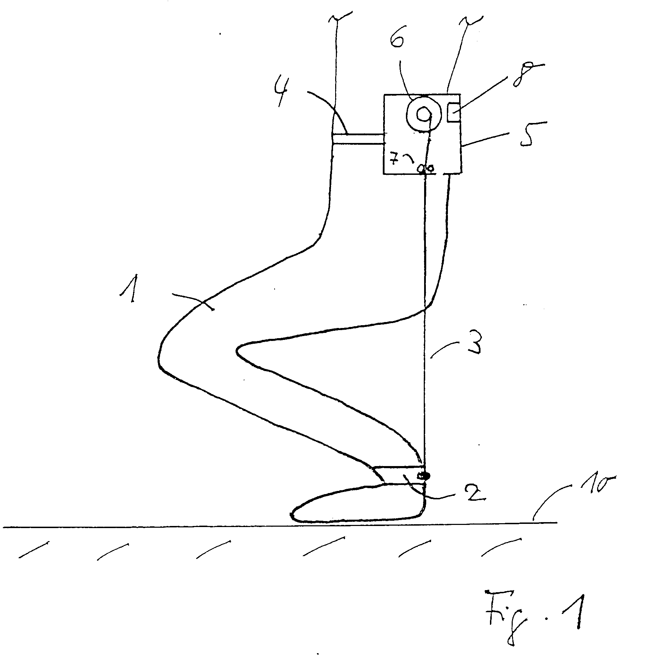

In Fig. 1 bezeichnet Bezugszeichen 1 die Beinpartie einer Testperson. Am Sprunggelenk der Testperson ist an einem Halteriemen 2, der zweckmässigerweise einen Haftverschluss oder einen Schnallenverschluss aufweist, ein Zugband 3 angebracht. Das Zugband 3 ist aus einem flexiblen, im wesentlichen undehnbaren Material, wie z.B. einem Metall oder Kunststoff, hergestellt. Anstattdessen kann auch ein Seil, ein Draht oder eine Kette mit entsprechenden Eigenschaften verwendet werden.

Das Zugband 3 ist ein Bestandteil der Messeinrichtung der ersten Ausführungsform des erfindungsgemässen Kniebeugen-Ergometers, deren weitere Bestandteile in einem Gehäuse 5 untergebracht sind, das mit einem Gürtel 4 im Hüftbereich der Testperson befestigbar ist.

Das Zugband 3 wird durch eine Gehäuseöffnung und ein im Gehäuse 5 vorgesehenes Führungsrollenpaar 7 möglichst reibungsfrei in das Gehäuse geführt. Die Gehäuseöffnung und das freie Ende des Zugbands 3 sind zweckmässigerweise derart gestaltet, dass das freie Ende des Zugbandes 3 im von der Testperson gelösten Zustand nicht in das Gehäuse 5 hineinschlupfen kann, sondern stets aus dem Gehäuse 5 herausschaut.

Innerhalb des Gehäuses 5 ist eine Spule 6 zum Auf- und Abwickeln des Zugbands 3 vorgesehen. Die zum Aufwickeln benötigte Zugkraft wird von einer nicht dargestellten Spiralfeder bereitgestellt, welche mit der Achse der Spule 6 in Verbindung steht.

Durch diesen Aufbau werden je ein unterhalb und ein oberhalb der Knie liegender Messpunkt definiert, nämlich einer am freien Ende des Zugbandes 3 liegender erster Meßpunkt, der am Sprunggelenk der Testperson befestigt ist, und ein im Gehäuse 5 fixierter zweiter Meßpunkt etwa im Zentrum der Spule 6, auf der das Zugband 3 aufgewickelt ist.

Im Gehäuse 5 ist weiterhin eine Sensoreinrichtung 8 vorgesehen, welche die mit dem Beugen und Strekken der Knie einhergehende Auf- und Abspulbewegungen des Zugbands 3 bezüglich der Spule 6 erfasst, nämlich die Längen der jeweils auf- und abgespulten Zugbandabschnitte und deren Auf- und Abspulgeschwindigkeit.

Eine in Fig. 1 nicht gezeigte mit der Sensoreinrichtung 8 verbundene Auswerteeinrichtung dient zur Speicherung eines ihr eingegebenen, der Testperson zugeordneten Gewichtswertes und zur Ermittlung der von der Testperson erbrachten Arbeit und / oder Leistung durch Verknüpfung des Gewichtswertes mit dem oder den von der Messeinrichtung gelieferten Werten.

Selbstverständlich können auch weitere auf die Testperson wirkende Gegenkräfte bei der Ermittlung der Arbeit und / oder Leistung berücksichtigt werden. Beispiele hierfür sind die Federkraft der Spiralfeder und mögliche Reibungskräfte.

Im allgemeinen Fall ergibt sich die gesamte erbrachte Arbeit durch Summation der Arbeitsanteile entlang kleiner Wegstrecken unter Berücksichtigung der auf diesen Wegstrecken wirkenden Kräfte. Diese Arbeitsanteile ergeben sich dabei durch Multiplikation der kleinen Wegstrecken mit den längs dieser kleinen Wegstrecken wirkenden Kräften.

Analog ergeben sich die entsprechenden Leistungsanteile durch Multiplikation der Geschwindigkeit auf den kleinen Wegstrecken, d.h. der pro Zeit zurückgelegten Wegstrecken, mit den längs dieser Wegstrecken wirkenden Kräften.

Der von der Auswerteeinrichtung benötigte Gewichtswert ist nicht gleich dem tatsächlichen Körpergewicht der Testperson, sondern - je nach Körperhaltung, insbesondere Beinhaltung, bei den Kniebeugen - nur ein Bruchteil davon.

Da es bei einem derartigen Trainingsgerät jedoch nicht auf einen äusserst exakten Absolutwert ankommt, sondern lediglich der relative Vergleich von Bedeutung ist, genügt es beispielsweise, die gesamte durch die Sensoreinrichtung 8 erfasste relevante Spulstrecke, also die gesamte erfasste Abstandsänderung, mit einem nach einer Faustformel ermittelten effektiven Gewichtswert zu multiplizieren.

Dieser effektive Gewichtswert könnte nach der Formel:

$$\text{effektiver Gewichtswert} = a \times \text{Körpergewicht}$$

wobei a eine Konstante kleiner als 1 ist, berechnet werden. Die Konstante a kann beispielsweise zwischen 0,5 und 1, bevorzugt zwischen 0,7 und 0,9 liegen.

Die gesamte erbrachte Arbeit und / oder Leistung oder auch nur gewisse Arbeitsanteile und / oder Leistungsanteile können auf einer (nicht dargestellten) Anzeigeeinrichtung, z.B. einer LCD-Anzeige oder einer Bildschirmeinrichtung, ausgegeben werden. Sie können auch über eine Schnittstelle an eine externe Auswerteeinrichtung weitergeleitet werden, welcher auch andere physische Daten der Testperson, z.B. dessen Pulsfrequenz o.ä., zugeführt werden. Zweckmässigerweise ist diese externe Auswerteeinrichtung ein Computer.

Der Betrieb des erfindungsgemässen Kniebeugen-Ergometers läuft folgendermassen ab. Nach Installation der Ergometereinrichtung in der in Fig. 1 gezeigten Art und Weise wird die Messeinrichtung zunächst initialisiert, d.h. es werden der Gewichtswert der Testperson eingegeben und die Resultatanzeige auf Null gestellt.

Daraufhin führt die auf dem Boden 10 stehende Testperson Kniebeugen durch, d.h. beugt und streckt ihre Beine gegen die Kraft ihres eigenen Körpergewichts. Dabei wird das Zugband 3 von der Spule 6 abwechselnd ab- und wieder aufgewickelt.

Die Länge des auf- bzw. abgewickelten Zugbands 3 - und auch die Auf- bzw. Abwickelgeschwindigkeit - werden durch die Sensoreinrichtung 8 erfasst und der Auswerteeinrichtung zugeführt und dort beitragsmässig summiert. Die Auswerteeinrichtung ermittelt hieraus, wie oben erwähnt, die verrichtete Arbeit und auch die dabei erbrachte Leistung der Testperson.

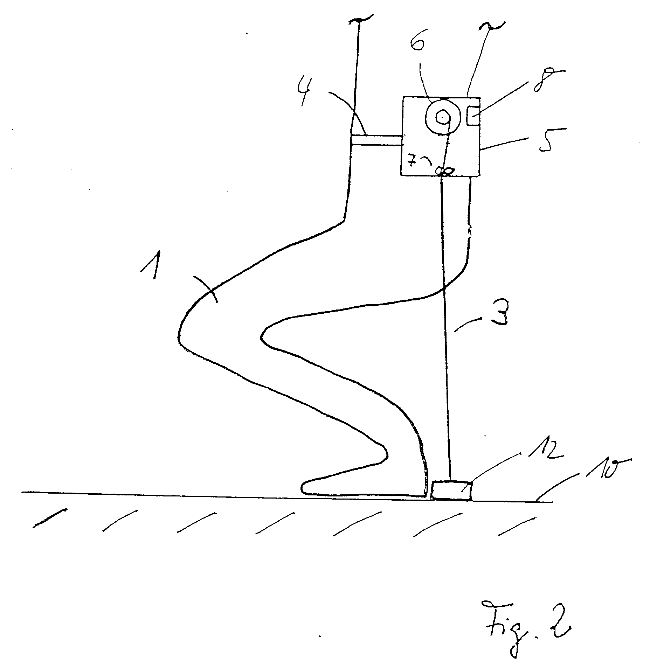

Bei der anhand Fig. 2 dargestellten zweiten Ausführungsform des erfindungsgemässen Kniebeugen-Ergometers ist das freie Ende des Zugbands 3 nicht am Körper der Testperson, sondern mit einer Befestigung 12 auf dem Boden 10, auf dem die Testperson steht, angebracht.

Durch diesen Aufbau werden ebenfalls zwei Messpunkte definiert, nämlich wiederum ein erster Messpunkt am freien Ende des Zugbandes 3, das hier zu Trainingszwecken am Boden 10 neben dem Fuss der Testperson befestigt ist, und ein zweiter Messpunkt an dergleichen Stelle, wie anhand Fig. 1 beschrieben.

Bei diesem Ausführungsbeispiel kann das Sprungvermögen der Testperson bei Kniestrecksprüngen ermittelt werden.

In den Darstellungen der Fig. 1 und Fig. 2 ist der eine Meßpunkt stets im Hüftbereich und der andere stets im Fussbereich der Testperson angeordnet worden. Die vorliegende Erfindung ist auf diese Meßpunktanordnung natürlich nicht beschränkt.

So kann das freie Ende des Zugbands 3 an jeder beliebigen Stelle des Körpers wie auch an jeder beliebigen Stelle getrennt vom Körper angebracht werden, um den ersten Messpunkt zu definieren. Das Gehäuse 5 kann ebenfalls an jeder beliebigen Stelle des Körpers angebracht werden, um den zweiten Messpunkt zu definieren. Nur müssen die Messpunktorte so gewählt sein, daß sich deren gegenseitiger Abstand beim Beugen / Strecken der Knie ändert.

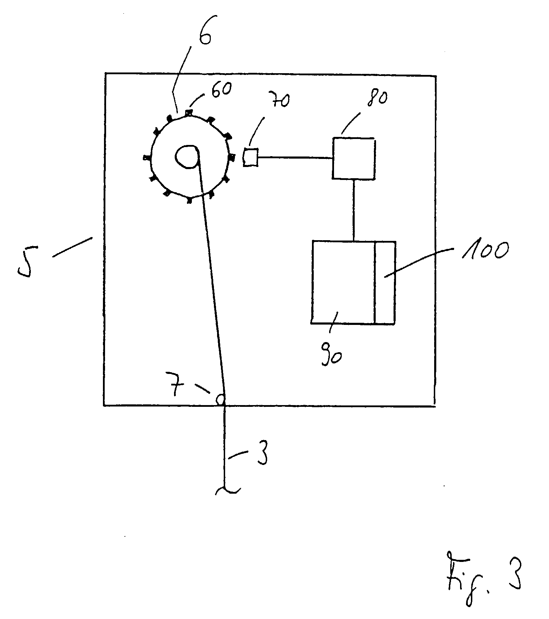

Fig. 3 zeigt eine detailliertere schematische Darstellung der Messeinrichtung des erfindungsgemässen Kniebeugen-Ergometers.

Gemäß Fig. 3 weist der Umfangsrand der Spule 6 Auskragungen in Form von Zähnen 60 aus einem magnetischen Material auf.

An einem festen Ort ist gegenüberliegend von den Zähnen 60 ein Induktionsdetektor 70 vorgesehen, in den jedesmal dann, wenn einer der Zähne 60 an ihm vorbeiläuft, eine Induktionsspannung induziert wird. Somit kann die Auf-und Abspulbewegung der Spule 60 inkremental erfasst werden. Der Drehsinn kann durch eine asymmetrische Form der Zähne 60 oder sonstige bei derartigen Inkrementalgebern übliche Techniken erfasst werden.

Das Impulssignal des Induktionsdetektors 70 wird dann über einen Analog-/Digital-Wandler 80 in ein entsprechendes digitales Signal gewandelt und an einen Mikrocomputer 90 geliefert, der nach Berechnung der Arbeit und / oder Leistung das Ergebnis auf einem Monitor 100 ausgibt.

Die Erfindung ist natürlich nicht auf derartige Inkrementalgeber beschränkt. So können beliebige Weg- bzw. Geschwindigkeitsmesseinrichtungen, etwa optische, akustische, optoelektronische etc., kombiniert oder separat verwendet werden, um die betreffende Abstandsänderung bzw. deren Geschwindigkeit zu bestimmen.

Auch kann anstelle der mit der Spule verbundenen elastischen Spiralfeder zum Auf- und Abrollen des Zugbandes eine andere Einrichtung zur abwechselnden Freigabe und Aufnahme von Zugband-Inkrementen verwendet werden, beipielsweise eine Gegengewichteinrichtung oder eine Falteinrichtung.

Schliesslich ist die vorliegende Erfindung nicht auf die Verwendung eines Zugbandes oder dergleichen beschränkt. Vielmehr können die Abstandsänderung und deren Geschwindigkeit auch verbindungslos über elektromagnetische - insbesondere optische -, akustische oder sonstige geeignete Wellen erfasst werden.

**Patentansprüche**

1. Kniebeugen-Ergometer mit:

   a) Befestigungsmitteln (2, 12; 4) zur Befestigung zweier vertikal beabstandeter Messpunkte an oder in Bezug auf eine(r) Testperson derart, daß der Meßpunktabstand sich durch Beugen / Strecken der Knie ändert;

   b) einer Messeinrichtung (3, 6, 8; 60, 70, 80), die ausgelegt ist:

      b.1) zur Messung der mit dem Beugen und Strecken der Knie einhergehenden Änderung des Abstandes zwischen den beiden Messpunkten und / oder

      b.2) zur Messung der Geschwindigkeit der im Merkmal b.1) genannten Abstandsänderung; und

   c) einer mit der Messeinrichtung (3, 6, 8; 60, 70, 80) verbundenen Auswerteeinrichtung (90), die ausgelegt ist:

      c.1) zur Speicherung eines ihr eingegebenen, der Testperson zugeordneten Gewichtswertes und

      c.2) zur Ermittlung der von der Testperson erbrachten Arbeit und / oder Leistung oder einer hierzu korrelierten Grösse durch Verknüpfung des Gewichtswertes mit dem oder den von der Messeinrichtung (3, 6, 8; 60, 70, 80) gelieferten Messwert(en).

2. Kniebeugen-Ergometer nach Anspruch 1, bei welchem die Befestigungsmittel (12) derart angeordnet sind, dass der erste Messpunkt unterhalb der Knie der Testperson liegt und der zweite Messpunkt oberhalb der Knie der Testperson liegt.

3. Kniebeugen-Ergometer nach Anspruch 2, bei welchem die Befestigungsmittel (12) an einem ersten Messpunkt, der getrennt vom Körper der Testperson, insbesondere am Fussboden (10) unter der Testperson, vorgesehen ist, befestigbar sind.

4. Kniebeugen-Ergometer nach Anspruch 2, bei welchem die Befestigungsmittel (2) an einem ersten Messpunkt, der am Körper der Testperson, insbesondere am Fuss oder Sprunggelenk der Testperson, vorgesehen ist, befestigbar sind.

5. Kniebeugen-Ergometer nach einem der vorhergehenden Ansprüche, bei welchem die Messeinrichtung (3, 6, 8; 60, 70, 80) eine nach Art eines Seiles, eines Bandes, einer Kette oder dergleichen ausgebildete Zugeinrichtung (3) aufweist, welche den ersten und den zweiten Messpunkt verbindet.

6. Kniebeugen-Ergometer nach Anspruch 5, bei welchem die Messeinrichtung (3, 6, 8; 60, 70, 80) eine Spuleneinrichtung (6) zum Auf- und Abspulen von zumindest einem Teil der Zugeinrichtung (3) aufweist.

7. Kniebeugen-Ergometer nach Anspruch 5 oder 6, bei welchem die Messeinrichtung (3, 6, 8; 60, 70, 80) eine Drehwinkel-Erfassungseinrichtung (8; 60, 70, 80) zum Erfassen des Drehwinkels und eine Drehwinkelgeschwindigkeits-Erfassungseinrichtung (8; 60, 70, 80) zum Erfassen der Drehwinkelgeschwindigkeit einer Umlenkeinrichtung für die Zugeinrichtung (3) aufweist.

8. Kniebeugen-Ergometer nach Anspruch 7, bei welchem die Umlenkeinrichtung die Spuleneinrichtung (6) ist.

9. Kniebeugen-Ergometer nach einem der Ansprüche 6 bis 8, bei welchem die Spuleneinrichtung (6) in einem Gehäuse (5) untergebracht ist, welches mittels der Befestigungsmittel (2, 12; 4) am Körper, insbesondere im Hüftbereich, der Testperson anbringbar ist.

10. Kniebeugen-Ergometer nach einem der vorhergehenden Ansprüche, bei welchem die Messeinrichtung (3, 6, 8; 60, 70, 80) eine optische oder eine elektronische Sensoreinrichtung (8; 60, 70, 80) zum Erfassen der Änderung des gegenseitigen Abstands der beiden Messpunkte und der Geschwindigkeit der Abstandsänderung aufweist.

11. Kniebeugen-Ergometer nach einem der vorhergehenden Ansprüche, bei welchem die Messeinrichtung (3, 6, 8; 60, 70, 80) mit einem Computer (90) verbunden ist.

12. Kniebeugen-Ergometer nach Anspruch 11, bei welchem der Computer (90) ein in dem am Körper der Testperson anbringbaren Gehäuse (5) untergebrachter Mikrocomputer ist.

Fig. 1

Fig. 2

Fig. 3

**Europäisches Patentamt**

# EUROPÄISCHER RECHERCHENBERICHT

Nummer der Anmeldung

EP 97 12 1871

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | EP 0 445 617 A (BOSCO CARMELO)<br>* Zusammenfassung *<br>* Spalte 2, Zeile 51 - Spalte 5, Zeile 34; Tabelle 1 *<br>--- | 1,10-12 | A61B5/22<br>A63B23/04<br>A63B21/00 |
| A | WO 94 26359 A (MULTIBUSINESS ENTERPRISE FINNT ;KAIKKONEN AUVO (FI); JAERVI HANNU)<br>* Seite 5, Zeile 1 - Seite 7, Zeile 9; Tabellen 1-3 *<br>--- | 1,7,11, 12 | |
| A | EP 0 430 067 A (COMBI CO)<br><br>* Spalte 4, Zeile 33 - Spalte 7, Zeile 37; Tabellen 1-6 *<br>----- | 1,5,6, 10-12 | |

| RECHERCHIERTE SACHGEBIETE (Int.Cl.6) |
|---|
| A61B<br>A63B |

Der vorliegende Recherchenbericht wurde für alle Patentansprüche erstellt

| Recherchenort | Abschlußdatum der Recherche | Prüfer |
|---|---|---|
| BERLIN | 11.Februar 1998 | Weihs, J |